## Europäisches Patentamt
## European Patent Office
## Office européen des brevets

(19)

(11) Veröffentlichungsnummer: **0 401 265 B1**

(12)
# EUROPÄISCHE PATENTSCHRIFT

(45) Veröffentlichungstag der Patentschrift: **11.05.94**

(51) Int. Cl.5: **A61N 1/365**

(21) Anmeldenummer: **89902756.9**

(22) Anmeldetag: **06.02.89**

(86) Internationale Anmeldenummer:
**PCT/EP89/00108**

(87) Internationale Veröffentlichungsnummer:
**WO 89/06990 (10.08.89 89/18)**

(54) **VERFAHREN ZUR ANPASSUNG DER STIMULATIONSFREOUENZ EINES HERZSCHRITTMACHERS AN DIE BELASTUNG EINES PATIENTEN.**

(30) Priorität: **05.02.88 DE 3803473**

(43) Veröffentlichungstag der Anmeldung:
**12.12.90 Patentblatt 90/50**

(45) Bekanntmachung des Hinweises auf die Patenterteilung:
**11.05.94 Patentblatt 94/19**

(84) Benannte Vertragsstaaten:
**DE FR GB IT NL SE**

(56) Entgegenhaltungen:
**EP-A- 0 059 868**
**US-A- 4 702 253**

(73) Patentinhaber: **SIEMENS AKTIENGESELL-SCHAFT**
**Wittelsbacherplatz 2**
**D-80333 München(DE)**

(84) Benannte Vertragsstaaten:
**DE FR GB IT NL**

(73) Patentinhaber: **Siemens Elema AB**
**Röntgenvägen 2**
**S-171 95 Solna 1(SE)**

(84) Benannte Vertragsstaaten:
**SE**

(72) Erfinder: **HEINZE, Roland**
**Albertus-Magnus-Weg 5**
**D-8012 Ottobrunn(DE)**

EP 0 401 265 B1

**Beschreibung**

Die Erfindung betrifft ein Verfahren zur Anpassung der Stimulationsfrequenz eines Herzschrittmachers an die Belastung eines Patienten, wobei ein Kennlinienregler die Stimulationsfrequenz in Abhängigkeit von einer belastungsabängigen Regelgröße regelt und wobei durch Änderung der Stimulationsfrequenz während bestimmter Zeitintervalle festgestellt wird, ob sich dabei ein vom Herzminutenvolumen abhängiger Meßwert ändert und wobei ein Optimierungsregler eine den Zusammenhang Regelgröße-Stimulationsfrequenz darstellende Kennlinie entsprechend dieser Meßwertänderung beeinflußt.

Ein Verfahren dieser Art ist aus der EP-A2-0 165 566 bekannt. Dabei wird die Stimulationsfrequenz eines Herzschrittmachers in Abhängigkeit von der zentralvenösen Blutsauerstoffsättigung im Herzen geregelt. Die zentralvenöse Blutsauerstoffsättigung wird nach dem Prinzip der Reflexionsoximetrie ermittelt, wie sie beispielsweise in der DE-A-31 52 963 näher beschrieben ist.

Mit einer fest vorgegebenen, unveränderbaren Kennlinie als Zusammenhang zwischen Blutsauerstoffsättigung und Stimulationsfrequenz ist keine optimale Anpassung an die individuelle und sich mit der Zeit ändernde hämodynamische Situation des Patienten möglich. Daher ist bei der genannten EP-A2-0 165 566 eine kontinuierlich neben der Kennlinienregelung wirkende optimierende Regelung vorgesehen. Die optimierende Regelung überwacht kontinuierlich die Tendenz des Blutsauerstoffsättigungswertes und stellt fest, ob durch selbständiges Erhöhen oder Erniedrigen der Frequenz eine Verbesserung (Meßwerterhöhung) des Blutsauerstoffsättigungswertes erfolgt oder nicht. Jedem Blutsauerstoffsättigungswert ist nicht mehr nur eine bestimmte, sondern eine ganze Schar von Kennlinien zugeordnet.

Diese Anordnung hat jedoch den Nachteil, daß sie nach jedem Frequenzwechsel eine Entscheidung trifft, ob eine positive oder negative Reaktion der Blutsauerstoffstättigung erfolgte, und damit die Gefahr von Fehlentscheidungen infolge störender, vor allem belastungsabhängiger Änderungen der Sauerstoffsättigung besteht.

Aufgabe der vorliegenden Erfindung ist es daher, ein Verfahren der eingangs genannten Art so auszugestalten, daß die optimale Anpassung der Stimulationsfrequenz-Regelung an die hämodynamische Situation des Patienten unabhängiger von Störeinflüssen und damit weiter verbessert wird.

Diese Aufgabe wird erfindungsgemäß dadurch gelöst, daß die Änderung der Stimulationsfrequenz während vom Herzschrittmacher vorgegebener Optimierungsintervalle mehrfach periodisch wiederholt wird und jeweils phasensynchron zur Stimulationsfrequenzänderung eine Erfassung des Herzminutenvolumenabhängigen Meßwertes erfolgt. Durch die mehrfache Wiederholung von Stimulationsfrequenzänderungen können Störeinflüsse weitgehend eliminiert werden.

Vorteilhafte Ausgestaltungen der Erfindung sind in den Unteransprüchen angegeben.

Ein Ausführungsbeispiel der Erfindung wird nachfolgend anhand der Figuren 1 bis 6 näher erläutert. Dabei zeigt:

FIG 1      die prinzipielle Anordnung eines Herzschrittmachers,
FIG 2      den Zusammenhang Stimulationsfrequenz - Herzmiflutenvolumen,
FIG 3      ein Blockschaltbild der Regelstrecke,
FIG 4      ein Blockschaltbild einer Regelschaltung,
FIG 5      eine Kennlinienschar Sauerstoffsättigung - Stimulationsfrequenz,
FIG 6      ein Impulsdiagramm.

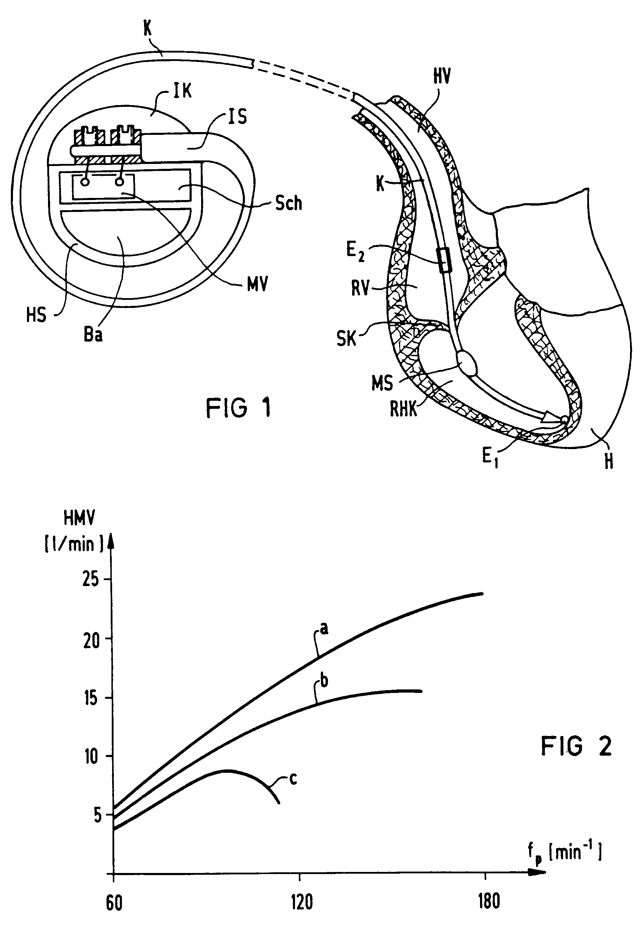

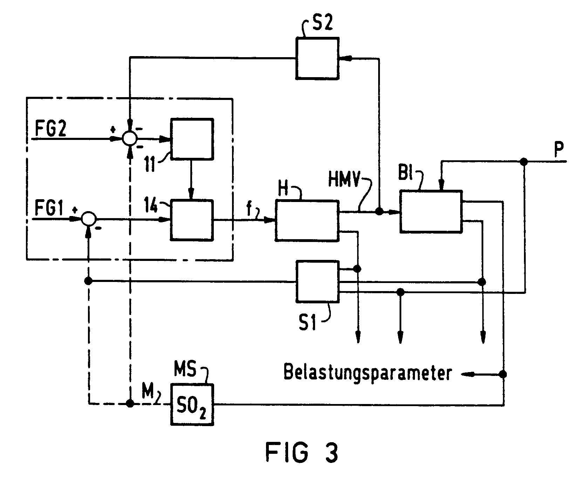

FIG 1 zeigt eine aus der DE-A-31 52 963 bekannte Anordnung eines Herzschrittmachers. Der Herzschrittmacher HS enthält einen Stromversorgungsteil Ba, einen elektronischen Schaltungsteil Sch mit dem Meßverstärker MV und die zweipolige elektrische Kupplung IK. In der Kupplung IK ist der zweipolige elektrische Stecker IS des Stimulationskatheters K festgeschraubt. Der Stimulationskatheter K führt über die obere Hohlvene HV in den rechten Vorhof RV, in dem die indifferente Sensingelektrode $E_2$ positioniert ist, und dann in die rechte Herzkammer RHK. Im Bereich der rechten Herzkammer RHK enthält der Stimulationskatheter K eine Meßsonde MS, die nach dem Prinzip der Reflexionsoximetrie die Blutsauerstoffsättigung mißt. Durch die Stimulationselektrode $E_1$ wird der Herzmuskel H gereizt, wobei die Stimulationsfrequenz vom Meßsignal der Meßsonde M, also von der Blutsauerstoffsättigung abhängig ist. Die Blutsauerstoffsättigung wirkt damit als Regelgröße für die Regelung der Stimulationsfrequenz.

Der Herzschrittmacher soll im Idealfall die Stimulationsfrequenz so einstellen, daß bei einer gleichbleibenden physischen Belastung die venöse Blutsauerstoffsättigung bei möglichst niedriger Frequenz möglichst hoch ist. Diese Aufgabenstellung rührt her von der Tatsache, daß die physiologische Anpassung des Herzmuskels an körperliche Belastungen P dann optimal ist, wenn das Herzminutenvolumen HMV proportional der körperlichen Belastung P ist: HMV $\,\hat{=}\,$ P. Um die Belastung des Herzmuskels zu optimieren, soll dabei ein bestimmtes Herzminutenvolumen mit möglichst großem Schlagvolumen $SV_{max}$ bei möglichst niedriger Pulsfrequenz $f_{pmin}$ erreicht werden, d.h.

$$HMV_{opt} \, (P = konstant) = \, SV_{max} \bullet f_{p \, min}$$

Weiterhin gilt: Bei gleichbleibender körperlicher Belastung (P = konstant), d.h. gleichbleibender Sauerstoffausschöpfung des Blutes im peripheren Kreislauf, ist die Änderung des venösen Blutsauerstoffs $\Delta SO_2$ proportional der Änderung des Herzminutenvolumens $\Delta HMV$. Auf der Basis der oben genannten Zusammenhänge soll der Herzschrittmacher aufgrund einer Optimalwertregelung eins an die Leistungsfähigkeit des Herzmuskels angepaßte Kennlinie aus einer Kennlinienschar Führungsgröße-Stimulationsfrequenz auswählen.

In FIG 2 ist der Zusammenhang zwischen der Herzfrequenz $f_p$ und dem Herzminutenvolumen HMV für ein gesundes Myokard (a) und zwei Fälle eines kranken Myokards (Kennlinien b,c) dargestellt. Aus den Kennlinien sieht man, daß das Herzminutenvolumen HMV im niedrigen Lastbereich etwa proportional zur Herzfrequenz $f_p$ steigt und dann je nach Leistungsfähigkeit des Myokards asymptotisch einen Grenzwert $HMV_{max}$ erreicht. Charakteristisch für das kranke Myokard ist der Abfall des Herzminutenvolumens HMV ab einer maximalen Herzfrequenz $f_{p \, max}$, weshalb eine Herzfrequenz größer als $f_{p \, max}$ als Stimulationsfrequenz auf jeden Fall zu vermeiden ist.

FIG 3 zeigt einen Regelkreis mit verschiedenen Möglichkeiten einer physiologischen Regelung der Stimulationsfrequenz. Dabei ist das Herz H als Stellglied zu betrachten, das über das Herzminutenvolumen HMV auf den Blutkreislauf BL wirkt. Der Blutkreislauf BL wird außerdem durch die als Störgröße betrachtete körperliche Belastung P beeinflußt.

Ein erster Sensor S1 ist zur Erfassung einer belastungsabhängigen physiologischen Größe, z.B. EKG, Blutdruck, Atemvolumen oder eines Aktivitätsmeßwerts der körperlichen Bewegung vorgesehen. Je nach erfaßter physiologischer Größe ist der Sensor S1 dazu wirkungsmäßig mit dem Herzen H, dem Blutkreislauf BL oder einer die körperliche Belastung P, z.B. die Aktivität, repräsentierenden Größe (P), verbunden.

Aus dem Ausgangssignal dieses Sensors S1 wird über ein Differenzglied, dessen zweitem Eingang eine erste Führungsgröße FG1 zugeführt ist, eine Regelgröße gebildet, die über den Regler 14 die Stimulationsfrequenz f für das Herz H vorgibt.

Ein Optimalregler 11 erhält als Regelsignal das über einen zweiten Sensor S2 erfaßte Herzminutenvolumen HMV, das von einer zweiten Führungsgröße FG2 subtrahiert wird. Dabei kann der Sensor S2 z.B. nach dem Prinzip der Impedanzmessung arbeiten. Der Optimalregler 11 regelt den vom Regler 14 vorgegebenen Zusammenhang Regelgröße - Stimulationsfrequenz auf einen optimalen Wert.

FIG 3 zeigt noch eine weitere - gestrichelt gezeichnete - Möglichkeit zur Optimalregelung mit nur einer Meßsonde MS für die Blutsauerstoffsättigung $SO_2$. Dabei wird der Meßwert für die Blutsauerstoffsättigung $SO_2$ sowohl dem Regler 14 als auch dem Optimalregler 11 als Regelgröße zugeführt. Die Sensoren S1 und S2 entfallen.

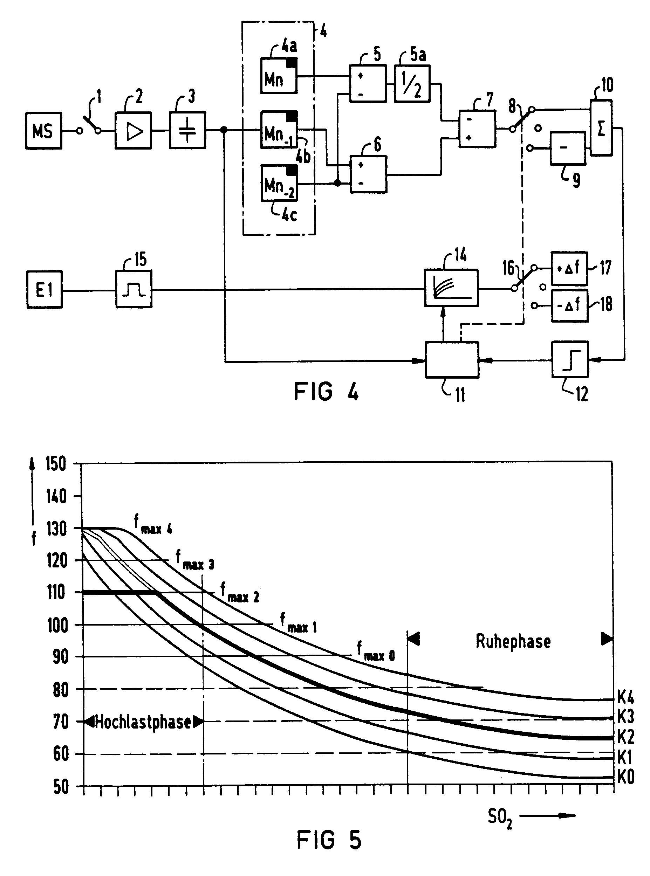

Diese wegen des Bedarfs von nur einer Meßsonde MS besonders vorteilhafte Ausführungsform wird im folgenden anhand des Ausführungsbeispiels nach FIG 4 näher erläutert.

FIG 4 zeigt ein Blockschaltbild einer Regelschaltung mit Optimierungsregelung. Dabei ist die Meßsonde MS über einen Schalter 1, einen Meßverstärker 2 und einen Mittelwertbildner 3 an einen Meßwertspeicher 4 angeschlossen. Der Meßwertspeicher 4 enthält Speicher 4a-4c für den jeweils aktuellen Meßwert $M_n$ und jeweils zwei vorausgehende Meßwerte $M_{n-1}$ und $M_{n-2}$.

Dem Meßwertspeicher 4 sind zwei Subtrahierglieder 5 und 6 nachgeschaltet, wobei das Subtrahierglied 5 den Meßwert $M_{n-2}$ vom Meßwert $M_n$ und das Subtrahierglied 6 den Meßwert $M_{n-2}$ vom Meßwert $M_{n-1}$ subtrahiert. Der Ausgang des Subtrahiergliedes 5 ist über ein Proportionalglied 5a mit dem Proportionalfaktor $\frac{1}{2}$ mit dem Minus-Eingang eines weiteren Subtrahiergliedes 7 und das Subtrahierglied 6 mit dem Plus-Eingang des Subtrahiergliedes 7 verbunden. Das Subtrahierglied 7 ist über einen Schalter 8 wahlweise direkt mit dem Eingang eines Summierers 10 für die in einer noch zu erläuternden Optimierungsphase erhaltenen Meßwerte, über einen Invertierer 9 mit demselben Summierer 10 oder mit einem freien Kontakt verbindbar. Der Schalter 8 wird von einer als Optimalregler wirkenden Steuerlogik 11 angesteuert. Der Ausgang des Summierers 10 ist über einen Komparator 12 mit einem Eingang der Steuerlogik 11 verbunden. Ferner wird die Steuerlogik 11 durch den am Ausgang des Mittelwertbildners 3 abgegriffenen Meßwert gesteuert. Die Steuerlogik 11 wirkt auf einen Regler 14 mit einem Kennliniengeber. Der Regler 14 regelt in Abhängigkeit von dem von der Steuerlogik 11 vorgegebenen Wert die Frequenz f eines Pulsgenerators 15, der mit der Stimulationselektrode E1 verbunden ist.

Die Kennlinie des Reglers 14 kann durch Anlegen verschiedener Konstantspannungen über einen Schalter 16 verschoben werden. In FIG 4 sind der Übersichtlichkeit halber nur zwei Konstantspannungselemente 17 und 18 sowie ein Umschalter 16 mit nur zwei Stellungen für die beiden Konstantspannungselemente 17 und 18 und einer Leerstellung angegeben.

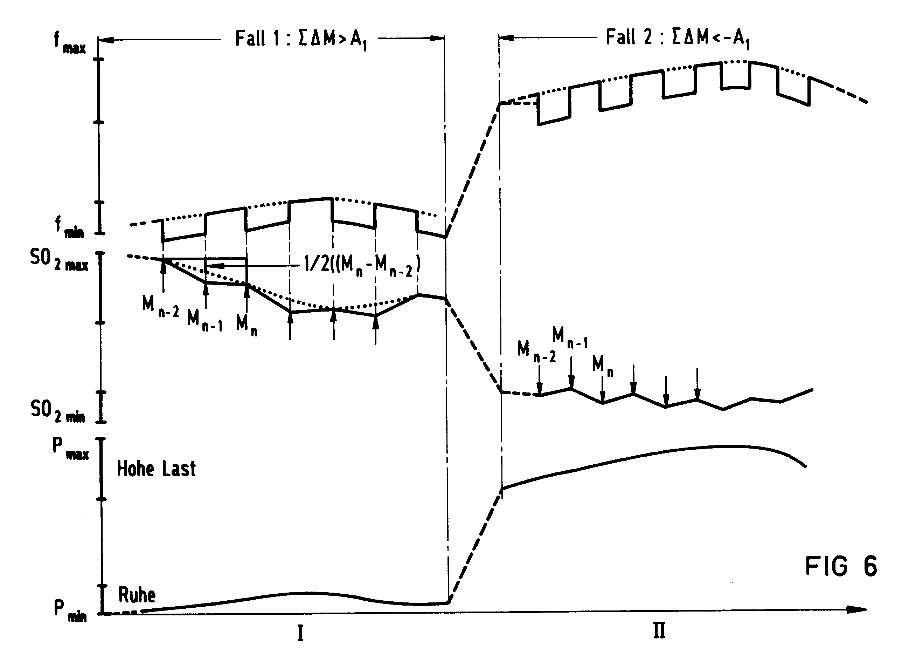

Die Wirkungsweise der Regelschaltung wird nachfolgend anhand der Figuren 5 und 6 näher erläutert.

FIG 5 zeigt eine Kennlinienschar für den Zusammenhang zwischen der normierten Blutsauerstoffsättigung $SO_{2\ norm}$ und der Stimulationsfrequenz f. Die Blutsauerstoffsättigung $SO_2$ wird mit der Meßsonde MS erfaßt, mit dem Meßwertverstärker 2 verstärkt, mit dem Mittelwertbildner 3 gemittelt und in der Steuerlogik 11 normiert. Im Kennliniengeber des Reglers 14 sind die Kennlinien K0-K4 abgespeichert, wobei mit dem Schalter 16 auf die verschiedenen Kennlinien umgeschaltet werden kann.

Ferner sind vier Maximalwerte $f_{max\ 0}$ - $f_{max\ 4}$ für die Stimulationsfrequenz festgelegt. Die Maximalwerte $f_{max\ 0}$ - $f_{max\ 4}$ werden durch die Steuerlogik festgelegt. Jede Kennlinie K0-K4 kann durch einen der Maximalwerte $f_{max\ 0}$ - $f_{max\ 4}$ beschnitten werden. Mit der Optimalregelung soll aus der in FIG 5 dargestellten Kennlinienschar eine an die Leistungsfähigkeit des Herzmuskels angepaßte Kennlinie K ausgewählt werden.

Dafür werden zunächst zwei charakteristische körperliche Zustände oder Phasen definiert, in denen eine individuelle Anpassung der Stimulationsfrequenz besonders wichtig ist, nämlich die Ruhephase und die Hochlastphase. Beide Phasen sind in FIG 5 eingezeichnet.

In der "Ruhephase" ist die durch einen Bereich hoher normierter Sauerstoffsättigung $SO_2$ erfaßte körperliche Aktivität auf ein Minimum reduziert. Es liegt eine geringe Sauerstoffausschöpfung vor und der zentralvenöse Wert der Sauerstoffsättigung erreicht sein Maximum.

In der "Hochlastphase" erreicht die durch einen Bereich niedriger normierter Sauerstoffsättigung $SO_{2\ norm}$ erfaßte körperliche Leistung eine individuell typische maximale Größe, d.h. es liegt eine maximale Sauerstoffausschöpfung vor bei einem minimalen zentralvenösen Wert der Sauerstoffsättigung $SO_2$.

Ideale Bedingungen für eine optimierende Frequenzanpassung wären gegeben, wenn damit in der Ruhephase eine zentralvenöse Sauerstoffsättigung $SO_2$ im Bereich zwischen 70 und 80 %, also ein Wert wie beim Herzgesunden, zu erreichen wäre und in der Hochlastphase ein für den Patienten maximales Herzminutenvolumen eingestellt werden könnte.

Voraussetzung für eine derartige Optimierung wäre allerdings eine Absolutwertmessung des Sauerstoffs und des Schlagvolumens. Diese Meßgrößen können mit den vorhandenen Methoden jedoch nicht direkt erfaßt werden. Für die Optimalregelung wird daher ein Meßverfahren benutzt, bei dem die genannten Meßgrößen indirekt erfaßt werden.

Die verwendete Optimierungsmethode beruht im Prinzip darauf, daß durch periodischen Wechsel der Stimulationsfrequenz und phasengleicher Meßwertauswertung darüber Informationen erhalten werden, ob und wie die Frequenzänderung die hämodynamische Situation, d.h. das Herzminutenvolumen des Patienten beeinflußt. Da das Herzminutenvolumen nicht direkt erfaßbar ist, wird als Ersatzgröße die vom Herzminutenvolumen abhängige Sauerstoffsättigung $SO_2$ verwendet.

Optimierungsintervalle werden, sofern die Optimierungsschaltung aktiviert ist, in regelmäßigen Abständen von z.B. sechs Stunden durchgeführt. Dabei wird eine Optimierung nur während der Ruhephase und während der Hochlastphase durchgeführt. Der Ablauf einer Optimierung wird im folgenden anhand des Diagramms nach FIG 6 näher erläutert.

FIG 6 zeigt einen angenommenen Belastungsverlauf P des Patienten für ein Optimierungsintervall I (Ruhephase) und ein z.B. einige Stunden später liegendes Optimierungsintervall II (Hochlastphase). Über der Belastung P ist gestrichelt die sich aufgrund einer vorgewählten Kennlinie K ergebende Stimulationsfrequenz f dargestellt. Ferner ist der Verlauf der Sauerstoffsättigung $SO_2$ dargestellt, wobei die gestrichelte Linie wieder den ohne Optimalregelung sich ergebenden Verlauf darstellt. Die Optimierung in der Ruhephase I startet mit einer Erniedrigung der Stimulationsfrequenz f, und zwar in der Ruhephase um z.B. 8 Schläge pro Minute. Diese Frequenzerniedrigung dauert z.B. 48 Herzschläge, und wird danach für 48 Herzschläge zurückgenommen und dann wiederholt. Um kurzzeitige Störeinflüsse - etwa durch Lastschwankungen -auszuschalten, werden je Optimierungsintervall z.B. 16 Frequenzwechsel durchgeführt und dann Störeinflüsse durch Summenbildung eliminiert.

Wie das Impulsdiagramm nach FIG 6 zeigt, wird synchron mit jedem Frequenzwechsel ein Meßwert $M_n$ der Sauerstoffsättigung $SO_2$ gespeichert. Die Meßwerte $M_n$ werden mit einer Zeitverzögerung (Hysterese) von 4 Schlägen gemessen, um die Verzögerung des Blutstromes im venösen Kreislauf zu berücksichtigen.

Zu jedem Meßwert $M_n$ werden im Meßwertspeicher 4 zwei vorangehende Meßwerte $M_{n-1}$ und $M_{n-2}$ abgespeichert.

Die Verrechnung der Meßwerte $\Delta M$ erfolgt nach jeder Wechselperiode, d.h. z.B. nach 96 Herzschlägen, wobei die festgestellte Meßwertdifferenz $\Delta M_n$ nach folgender Gleichung bestimmt wird:

$\Delta M = (M_{n-1} - M_{n-2}) - \frac{1}{2}(M_n - M_{n-2})$ bei Erhöhung der Stimulationsfrequenz

$\Delta M = -(M_{n-1} - M_{n-2}) + \frac{1}{2}(M_n - M_{n-2})$ bei Erniedrigung der Stimulationsfrequenz

Würde man einfach die Differenz zwischen zwei aufeinanderfolgenden Meßwerten verwenden, so hätte man damit auch den Einfluß von Belastungsänderungen, die den punktierten Verlauf der Sauerstoffsättigung $SO_2$ ergeben, erfaßt. Es soll jedoch nur der Einfluß der Frequenzänderung ermittelt werden. Wenn man annimmt, daß der Verlauf der Sauerstoffsättigung $SO_2$ ohne die periodische Stimulationsfrequenzänderung linear ist, so erhält man beim Meßwert $M_{n-1}$ eine von der periodischen Frequenzänderung unabhängige Änderung der Sauerstoffsättigung von $\frac{1}{2} (M_n - M_{n-2})$. Wenn man bei einer Erhöhung der Stimulationsfrequenz diesen Faktor $\frac{1}{2} (M_n - M_{n-2})$ von der gesamten Meßwertänderung $(M_{n-1} - M_{n-2})$ subtrahiert bzw. bei einer Erniedrigung der Stimulationsfrequenz f diesen Wert addiert, so erhält man mit guter Näherung die durch die periodische Änderung der Stimulationsfrequenz verursachte Änderung $\Delta M$ des Meßwertes für die Sauerstoffsättigung.

Wie bereits erwähnt, werden bevorzugt z.B. 16 Differenzmessungen nacheinander durchgeführt, wobei in FIG 6 der Übersichtlichkeit wegen nur ein Teil der Frequenzwechsel dargestellt ist. Eine Kombination aller 16 Meßwertänderungen $\Delta M$ wird dann zur Optimierungsregelung herangezogen.

Dabei könnte z.B. eine der beispielsweise aus Profos, Handbuch der industriellen Meßtechnik 1978, Seiten 185 bis 189 bekannten Korrelations funktionen herangezogen werden. Im beschriebenen Ausführungsbeispiel wird jedoch die Summe $\Sigma \Delta M$ der Meßwertänderungen ausgewertet, indem die am Ausgang des Summierers 7 anstehenden Meßwerte $\Delta M$ dem Summierglied 10 zugeführt werden.

Bei der Summenbildung der Meßwertänderungen $\Delta M$ müssen selbstverständlich die bei Frequenzerniedrigungen erhaltenen Meßwertänderungen $\Delta M$ mit dem inversen Vorzeichen der bei Frequenzerhöhungen erhaltenen Meßwertänderungen $\Delta M$ bewertet werden, was durch den Invertierer 9 erfolgt.

Die Ermittlung der Meßwertänderungen wird in analoger Weise während eines Optimierungsintervalls II in einer Hochlastphase durchgeführt. In der Hochlastphase kann die Änderung der Stimulationsfrequenz mit z.B. 16 pro Minute höher gewählt werden als in der Ruhephase. Auch in der Hochlastphase erhält man nach 16 Differenzmessungen durch Summenbildung eine für die Optimierungsregelung verwendete Meßwertänderung $\Delta M$.

Es ist möglich, daß in einem Optimierungszyklus I bzw. II nicht die vorgesehene Zahl von Meßwertänderungen $\Delta M$, im Ausführungsbeispiel also 16, gewonnen werden kann, weil z.B. inzwischen die Belastung P des Patienten wechselt. In diesem Fall werden die bereits gewonnenen Meßwertänderungen $\Delta M$ abgespeichert und für den nächsten Optimierungszyklus verwendet.

Die nach Durchführung der besprochenen Meßwerterfassung am Ausgang des Summierers 10 anstehenden Meßwerte $\Sigma \Delta M$ werden nun im Komparator 12 mit zwei Schwellwerten $-A_1$ und $+A_1$ verglichen. In Abhängigkeit vom Vergleichsergebnis wird gemäß der Tabelle auf Seite 13 aus der Kennlinienschar nach FIG 5 die optimale Kennlinie ausgewählt. Dabei wird in der Ruhephase ausschließlich die Kennlinie K bestimmt, während in der Hochlastphase die maximale Stimulationsfrequenz $f_{max}$ festgelegt wird. Wenn man annimmt, daß im Kennliniengeber des Reglers 14 zunächst die in FIG 5 verstärkt dargestellte Kennlinie 2 und die maximale Stimulationsfrequenz $f_{max\ 2}$ eingestellt war; so wird also beispielsweise die Kennlinie K1 ausgewählt, wenn die Summe der Meßwertänderungen $\Sigma \Delta M$ in mindestens einem Optimierungsintervall $<-A_1$ ist, d.h. also, wenn durch Reduzierung der Stimulationsfrequenz f eine Verbesserung der Sauerstoffsättigung erzielt wurde. Dagegen wird die Kennlinie K3 ausgewählt, wenn die Summe der Meßwertänderungen in einem Optimierungsintervall I, d.h. in der Ruhephase, $> A_1$ ist. Das bedeutet, daß durch Erhöhung der Stimulationsfrequenz eine Verbesserung der Sauerstoffsättigung $SO_2$ erzielt wurde, so daß die Kennlinie K3 für den betreffenden Patienten besser geeignet erscheint.

Wenn in einem Optimierungsintervall I in der Ruhephase die Summe der Meßwertänderungen zwischen $-A_1$ und $+A_1$ liegt, läßt sich daraus schließen, daß eine Änderung der Stimulationsfrequenz keinen wesentlichen Einfluß auf die Blutsauerstoffsättigung $SO_2$ hat. Die Kennlinie K2 wird somit zunächst beibehalten. Wenn jedoch die Summe der Meßwertänderungen $\Sigma \Delta M$ in zwei aufeinanderfolgenden Optimierungsintervallen I zwischen $-A_1$ und $+A_1$ liegt, wird die Kennlinie K1 aktiviert. Dies rührt von dem Bestreben her, in Zweifelsfällen stets niedrigeren Stimulationsfrequenzen den Vorrang zu geben, da dadurch der Herzmuskel geschont wird. Sollte sich durch den Übergang auf die Kennlinie K1 eine Verschlechterung der Sauerstoffsättigung ergeben, so wird im nächsten Optimierungsintervall I die Summe der Meßwertänderungen $\Sigma \Delta M$ wieder $> -A_1$ sein und damit wieder die Kennlinie K2 ausgewählt.

Wenn allerdings die Kennlinie K0, d.h. die untere Grenz-Kennlinie aktiviert ist und in zwei aufeinanderfolgenden Optimierungsintervallen I die Summe der Meßwertänderungen $\Sigma \Delta M$ zwischen $-A_1$ und $+A_1$ liegt, wird auf die Kennlinie K1 übergegangen. Damit wird verhindert, daß die Optimierungsregelung auf der unteren Grenz-Kennlinie K0 hängenbleibt.

Analoge Maßnahmen gemäß der dargestellten Tabelle werden auch für den Fall eines Optimierungsintervalls II für die Hochlastphase durchgeführt. Es besteht lediglich der Unterschied, daß hierbei nicht die Kennlinien K als solche beeinflußt werden, sondern die Grenzwerte $f_{max}$ für die Stimulationsfrequenz f.

Für die Konstanten A1 und A2 können verschiedene Werte für die Ruhepause und die Hochlastphase vorgegeben werden in Abhängigkeit von der Sensitivität der Meßsonde MS und der hämodynamischen Reserve des Patienten.

Tabelle

| Kombination | Bedingung | eingestellte Kennlinie bzw. Grenzfrequenz | Änderung bei | |
|---|---|---|---|---|
| | | | Ruhephase | hoher Lastphase |
| 1 | $1\ mal < A_1$ | 1-4 | Erniedrigen auf nächste Kennlinie (K0-K3) | Erniedrigen Grenzfrequenz um eine Stufe ($f_{max\ 0}$-$f_{max\ 3}$) |
| 2 | $1\ mal < A_1\ und > -A_1$ | 0-4 | Kennlinie bleibt | Grenzfrequenz bleibt |
| 3 | $2\ mal < A_1\ und > -A_1$ | 1-4 | Erniedrigen auf nächste Kennlinie (K0-K3) | Erniedrigen Grenzfrequenz um eine Stufe ($f_{max\ 0}$-$f_{max\ 3}$) |
| 4 | $2\ mal < A_1\ und > -A_1$ | 0 | Erhöhen auf nächste Kennlinie (K1) | Erhöhen Grenzfrequenz um eine Stufe ($f_{max\ 1}$) |
| 5 | $1\ mal > A_1$ | 0-3 | Erhöhen auf Kennlinie (K1-K4) | Erhöhen Grenzfrequenz um eine Stufe ($f_{max\ 1}$-$f_{max\ 4}$) |

**Patentansprüche**

1. Verfahren zur Anpassung der Stimulationsfrequenz (f) eines Herzschrittmachers an die Belastung eines Patienten, wobei ein Kennlinienregler (14) die Stimulationsfrequenz (f) in Abhängigkeit von einer belastungsabhängigen Regelgröße regelt und wobei durch Änderung der Stimulationsfrequenz (f) während bestimmter Zeitintervalle ($\Delta t$) festgestellt wird, ob sich dabei ein vom Herzminutenvolumen (HMV) abhängiger Meßwert (M) ändert und wobei ein Optimierungsregler (11) eine den Zusammenhang Regelgröße - Stimulationsfrequenz (f) darstellende Kennlinie entsprechend dieser Meßwertänderung ($\Delta M$) beeinflußt,
**dadurch gekennzeichnet,** daß die Änderung der Stimulationsfrequenz (f) während vom Herzschrittmacher (H) vorgegebener Optimierungsintervalle (I, II) mehrfach periodisch wiederholt wird und jeweils phasensynchron zur Stimulationsfrequenzänderung ($\Delta f$) eine Erfassung des Herzminutenvolumen-abhängigen Meßwertes (M) erfolgt.

2. Verfahren nach Anspruch 1, **dadurch gekennzeichnet,** daß alle Meßwerte (M(t)), die während einer Frequenzänderungsperiode ($\Delta t$) erfaßt werden, nach an sich bekannten Korrelationsverfahren mit Meßwerten (M(t $-n\Delta t$) der vorhergegangenen Frequenzänderungsperioden verrechnet werden und daß das Ergebnis zur Einstellung der Kennlinie belastungsabhängige Regelgröße - Stimulationsfrequenz (f) benutzt wird.

3. Verfahren nach Anspruch 1, **dadurch gekennzeichnet,** daß die Summe der während eines Optimierungsintervalles (I,II) erhaltenen Meßwertänderungen ($\Delta M$) verrechnet und das Ergebnis zur Einstellung der Kennlinie belastungsabhängige Regelgröße (M)-Stimulationsfrequenz (f) benutzt wird.

4. Verfahren nach einem der Ansprüche 1 bis 3, **dadurch gekennzeichnet,** daß positive und negative Stimulationsfrequenzänderungen ($\Delta f$) ausgewertet werden, wobei die sich daraus ergebenden Meßwert-änderungen ($\Delta M$) vorzeichenrichtig bewertet werden.

5. Verfahren nach einem der Ansprüche 1 bis 4, **dadurch gekennzeichnet,** daß zu jedem aktuellen Meßwert ($M_n$) mindestens zwei bei vorausgehenden Stimulationsfrequenzänderungen ($\Delta f$) erfaßte Meßwerte ($M_{n-1}$, - $M_{n-2}$) abgespeichert werden und daß die Meßwertänderung $\Delta M$ abhängig vom Vorzeichen der aktuellen Stimulationsfrequenzänderung ($\Delta f$) nach folgender Gleichung berechnet wird:
bei Erhöhung der Stimulationsfrequenz (f)

$$\Delta M = (M_{n-1} - M_{n-2}) - \tfrac{1}{2}(M_n - M_{n-2})$$

bei Erniedrigung der Stimulationsfrequenz (f)

$$\Delta M = -(M_{n-1} - M_{n-2}) + \tfrac{1}{2}(M_n - M_{n-2})$$

6. Verfahren nach einem der Ansprüche 1 bis 5, **dadurch gekennzeichnet,** daß die Summe der Meßwertänderungen $\Delta M$ in einem Optimierungsintervall (I,II) mit einem oberen positiven Grenzwert (A1) und einem unteren negativen Grenzwert (-A1) verglichen wird und daß die Kennlinie: Regelgröße ($SO_2$) - Stimulationsfrequenz (f) nach folgendem Schema verändert wird:

a) die Kennlinie (K) Regelgröße ($SO_2$) - Stimulationsfrequenz (f) wird nach niedrigeren Stimulationsfrequenzen (f) verschoben, wenn die Summe der Meßwertänderungen ($\Delta M$) in mindestens einem Optimierungsintervall (I,II) kleiner als -A1 ist;

b) die Kennlinie (K) Regelgröße - Stimulationsfrequenz (f) bleibt unverändert, wenn die Summe der Meßwertänderungen ($\Delta M$) zwischen (-A1) und (+A1) liegt;

c) die Kennlinie (K) Regelgröße - Stimulationsfrequenz (f) wird in Richtung höherer Stimulationsfrequenzen (f) verschoben, wenn die Summe der Meßwertänderungen ($\Delta M$) in mindestens einem Optimierungsintervall größer als A1 ist.

7. Verfahren nach Anspruch 6, **dadurch gekennzeichnet,** daß die Kennlinie in Richtung niedrigerer Stimulationsfrequenzen (f) verschoben wird, wenn die Summe der Meßwertänderungen ($\Delta M$) in zwei aufeinanderfolgenden Optimierungsintervallen (I,II) zwischen -A1 und +A1 liegt.

8. Verfahren nach Anspruch 6 oder 7, **dadurch gekennzeichnet,** daß die Kennlinie (K) in Richtung höherer Stimulationsfrequenzen (f) verschoben wird, wenn die Summe der Meßwertänderungen ($\Delta M$) in zwei aufeinanderfolgenden Optimierungsintervallen (I,II) zwischen -A1 und +A1 liegt und eine untere Grenz-Kennlinie (K0) erreicht ist.

9. Verfahren nach einem der Ansprüche 6 bis 8, **dadurch gekennzeichnet,** daß die dort dargestellte Kennlinienverschiebung nur in einer Ruhephase des Patienten durchgeführt wird.

10. Verfahren nach einem der Ansprüche 1 bis 9, **dadurch gekennzeichnet,** daß die Summe der Meßwertänderungen ($\Delta M$) mit einem oberen positiven Grenzwert (A1) und einem unteren negativen Grenzwert (-A1) verglichen wird und daß während einer hohen Belastungsphase des Patienten der obere Grenzwert der Stimulationsfrequenz ($f_{max}$) nach folgendem Schema verändert wird:

a) der Grenzwert ($f_{max}$) wird reduziert, wenn die Summe der Meßwertänderungen ($\Delta M$) in mindestens einem Optimierungsintervall kleiner als (-A1) ist und der Grenzwert ($f_{max}$) noch nicht seinen untersten vorgegebenen Wert erreicht hat;

b) der Grenzwert ($f_{max}$) bleibt unverändert, wenn die Summe der Meßwertänderungen ($\Delta M$) zwischen (-A1) und (+A1) liegt;

c) der Grenzwert ($f_{max}$) wird erhöht, wenn die Summe der Meßwertänderungen ($\Delta M$) in mindestens einem Optimierungsintervall größer als A1 ist und der Grenzwert ($f_{max}$) noch nicht seinen obersten vorgegebenen Wert erreicht hat.

11. Verfahren nach Anspruch 10, **dadurch gekennzeichnet**, daß der Grenzwert ($f_{max}$) reduziert wird, wenn die Summe der Meßwertänderungen ($\Delta M$) in zwei aufeinanderfolgenden Optimierungsintervallen (II) zwischen (-A1) und (+A1) liegt und der Grenzwert ($f_{max}$) noch nicht seinen untersten vorgegebenen Wert erreicht hat.

12. Verfahren nach den Ansprüchen 10 und 11, **dadurch gekennzeichnet**, daß der Grenzwert ($f_{max}$) erhöht wird, wenn die Summe der Meßwertänderungen ($\Delta M$) in zwei aufeinanderfolgenden Optimierungsintervallen (II) zwischen (-A1) und (+A1) liegt und der Grenzwert ($f_{max}$) seinen untersten vorgegebenen Wert erreicht hat.

13. Verfahren nach einem der Ansprüche 1 bis 12, **dadurch gekennzeichnet,** daß bei Optimierungsintervallen (I,II), in denen nicht die für einen Optimierungsvorgang ausreichende Zahl von Meßwertänderungen ($\Delta M$) gewonnen werden kann, die gemessenen Meßwertänderungen abgespeichert und für das nächste Optimierungsintervall weiterverwendet werden.

**Claims**

1.  Method for adapting the stimulation frequency (f) of a heart pacemaker to the loading of a patient, wherein a characteristic controller (14) controls the stimulation frequency (f) in dependence upon a load-dependent controlled variable and wherein, by changing the stimulation frequency (f) during certain time intervals (Δt), it is ascertained whether in the process a measured value (M) dependent on the cardiac minute output (HMV) changes and wherein an optimization controller (11) influences a characteristic representing the correlation of controlled variable and stimulation frequency (f) corresponding to this measured-value change (ΔM), characterized in that the change of the stimulation frequency (f) during optimization intervals (I, II) specified by the heart pacemaker (H) is periodically repeated several times and in each case phase-synchronous to the stimulation-frequency change (Δf) a detection of the measured value (M) dependent on the cardiac minute output takes place.

2.  Method according to claim 1, characterized in that all measured values (M(t)), which are detected during a frequency-change period (Δt), are reckoned up, according to correlation methods known in themselves, with measured values (M(t -nΔt) of the preceding frequency-change periods and in that the result is used for adjusting the characteristic of the load-dependent controlled variable and the stimulation frequency (f).

3.  Method according to claim 1, characterized in that the sum of the measured-value changes (ΔM) obtained during an optimization interval (I,II) is reckoned up and the result is used for adjusting the characteristic of the load-dependent controlled variable (M) and the stimulation frequency (f).

4.  Method according to one of claims 1 to 3, characterized in that positive and negative stimulation-frequency changes (Δf) are evaluated, whereby the measured-value changes (ΔM) resulting therefrom are evaluated correctly in terms of sign.

5.  Method according to one of claims 1 to 4, characterized in that for each actual measured value ($M_n$) at least two measured values ($M_{n-1}$, $M_{n-2}$) detected with preceding stimulation-frequency changes (Δf) are stored and in that the measured-value change ΔM dependent on the sign of the actual stimulation-frequency change (Δf) is calculated according to the following equation:
    with rising of the stimulation frequency (f)

    $$\Delta M = (M_{n-1} - M_{n-2}) - \tfrac{1}{2}(M_n - M_{n-2})$$

    with lowering of the stimulation frequency (f)

    $$\Delta M = -(M_{n-1} - M_{n-2}) + \tfrac{1}{2}(M_n - M_{n-2})$$

6.  Method according to one of claims 1 to 5, characterized in that the sum of the measured-value changes ΔM in an optimization interval (I,II) is compared with an upper positive limiting value (A1) and a lower negative limiting value (-A1) and in that the characteristic of controlled variable ($SO_2$) and stimulation frequency (f) is altered according to the following scheme:
    a) the characteristic (K) of the controlled variable ($SO_2$) and the stimulation frequency (f) is displaced towards lower stimulation frequencies (f) if the sum of the measured-value changes (ΔM) in at least one optimization interval (I,II) is smaller than -A1;
    b) the characteristic (K) of controlled variable and stimulation frequency (f) remains unchanged if the sum of the measured-value changes (ΔM) lies between (-A1) and (+A1);
    c) the characteristic (K) of controlled variable and stimulation frequency (f) is displaced in the direction of higher stimulation frequencies (f) if the sum of the measured-value changes (ΔM) in at least one optimization interval is greater than A1.

7.  Method according to claim 6, characterized in that the characteristic is displaced in the direction of lower stimulation frequencies (f) if the sum of the measured-value changes (ΔM) in two successive optimization intervals (I,II) lies between -A1 and +A1.

8.  Method according to claim 6 or 7, characterized in that the characteristic (K) is displaced in the direction of higher stimulation frequencies (f) if the sum of the measured-value changes (ΔM) in two

EP 0 401 265 B1

successive optimization intervals (I,II) lies between -A1 and + A1 and a lower limiting characteristic (KO) is achieved.

9.  Method according to one of claims 6 to 8, characterized in that the characteristic displacement represented there is only carried out in a resting phase of the patient.

10. Method according to one of claims 1 to 9, characterized in that the sum of the measured-value changes ($\Delta M$) is compared with an upper positive limiting value (A1) and a lower negative limiting value (-A1) and in that during a high load phase of the patient the upper limiting value of the stimulation frequency ($f_{max}$) is changed according to the following scheme:
   a) the limiting value ($f_{max}$) is reduced if the sum of the measured-value changes ($\Delta M$) in at least one optimization interval is less than (-A1) and the limiting value ($f_{max}$) has not yet reached its lowest specified value;
   b) the limiting value ($f_{max}$) remains unchanged if the sum of the measured-value changes ($\Delta M$) lies between (-A1) and (+A1);
   c) the limiting value ($f_{max}$) is increased if the sum of the measured-value changes ($\Delta M$) in at least one optimization interval is greater than A1 and the limiting value ($f_{max}$) has not yet reached its uppermost specified value.

11. Method according to claim 10, characterized in that the limiting value ($f_{max}$) is reduced if the sum of the measured-value changes ($\Delta M$) in two successive optimization intervals (II) lies between (-A1) and (+A1) and the limiting value ($f_{max}$) has not yet reached its lowest specified value.

12. Method according to claims 10 and 11, characterized in that the limiting value ($f_{max}$) is increased if the sum of the measured-value changes ($\Delta M$) in two successive optimization intervals (II) lies between (-A1) and (+A1) and the limiting value ($f_{max}$) has reached its lowest specified value.

13. Method according to one of claims 1 to 12, characterized in that with optimization intervals (I,II), in which the number of measured-value changes ($\Delta M$) adequate for an optimization procedure cannot be obtained, the measured measured-value changes are stored and further used for the next optimization interval.

**Revendications**

1.  Procédé pour adapter la fréquence de stimulation (f) d'un stimulateur cardiaque à l'effort d'un patient, selon lequel un régulateur (14) de courbe caractéristique règle la fréquence de stimulation (f) en fonction d'une grandeur de régulation qui dépend de l'effort, et selon lequel moyennant une modification de la fréquence de stimulation (f) pendant un intervalle de temps ($\Delta t$) déterminé, on détermine si une valeur de mesure (M), qui dépend du volume du débit minute du coeur (HMV) varie, et selon lequel un régulateur d'optimisation (11) influe sur une courbe caractéristique représentant la relation grandeur de régulation - fréquence de stimulation (f), en fonction de cette variation ($\Delta M$) de la valeur de mesure, caractérisé par le fait que la modification de la fréquence de stimuiation (f) est répétée périodiquement à plusieurs reprises pendant des intervalles d'optimisation (I, II), prédéterminés par le stimulateur cardiaque (H), et qu'une détection de la valeur de mesure (M), qui dépend du débit minute du coeur, s'effectue respectivement en synchronisme de phase avec la modification ($\Delta f$) de la fréquence de stimulation.

2.  Procédé suivant la revendication 1, caractérisé par le fait que toutes les valeurs de mesure (M(e)), qui sont détectées pendant une période ($\Delta t$) de modification de la fréquence, sont calculées selon des procédés de corrélation connus en soi, avec des valeurs de mesure (M(t-n$\Delta t$)) des périodes précédentes de variation de la fréquence et que le résultat est utilisé pour régler la courbe caractéristique grandeur de régulation - fréquence de stimulation (f) en fonction de l'effort.

3.  Procédé suivant la revendication 1, caractérisé par le fait qu'on calcule la somme des variations ($\Delta M$) de la valeur de mesure, obtenues pendant un intervalle d'optimisation (I, II) et qu'on utilise le résultat pour régler la courbe caractéristique grandeur de régulation (m) - fréquence de stimulation (f) en fonction de l'effort.

9

**4.** Procédé suivant l'une des revendications 1 à 3, caractérisé par le fait qu'on évalue des variations positive et négative ($\Delta$f) de la fréquence de stimulation, ce qui permet d'évaluer, d'une manière correcte du point de vue du signe, les variations ($\Delta$M), qui en résultent, de la valeur de mesure.

**5.** Procédé suivant l'une des revendications 1 à 4, caractérisé par le fait que pour chaque valeur actuelle ($M_n$),au moins deux valeurs de mesure ($M_{n-1}$, $M_{n-2}$), détectées dans le cas de modifications précédentes ($\Delta$f) de la fréquence de stimulation, sont mémorisées et que la variation ($\Delta$) de la valeur de mesure est calculée en fonction du signe de la variation actuelle ($\Delta$f) de la fréquence de stimulation, conformément à l'équation suivante :

lors d'un accroissement de la fréquence de stimulation (f)

$$\Delta M = (M_{n-1} - M_{n-2}) - \tfrac{1}{2} (M_n - M_{n-2})$$

lors d'une réduction de la fréquence de stimulation (f)

$$\Delta M = (M_{n-1} - M_{n-2}) + \tfrac{1}{2} (M_n - M_{n-2})$$

**6.** Procédé suivant l'une des revendications 1 à 5, caractérisé par le fait que la somme des variations ($\Delta$M) de la valeur de mesure pendant un intervalle d'optimisation (I, II) est comparée à une valeur limite supérieure positive (A1) et à une valeur limite inférieure négative (-A1), et que la courbe caractéristique : grandeur de régulation ($SO_2$) - fréquence de stimulation (f) est modifiée selon le schéma suivant :
a) la courbe caractéristique (K) grandeur de régulation ($SO_2$) - fréquence de stimulation (f) est décalée vers des fréquences de stimulation plus basses (f) lorsque la somme des variations ($\Delta$M) de la valeur de mesure dans au moins un intervalle d'optimisation (I, II) est inférieure à -A1;
b) la courbe caractéristique (K) grandeur de régulation - fréquence de stimulation (f) reste inchangée, lorsque la somme des variations ($\Delta$M) de la valeur de mesure se situe entre -A1 et +A1;
c) la courbe caractéristique (K) grandeur de régulation - fréquence de stimulation (f) est décalée en direction de fréquences supérieures de stimulation (f), lorsque la somme des variations ($\Delta$M) de la valeur de mesure dans au moins un intervalle d'optimisation est supérieure à A1.

**7.** Procédé suivant la revendication 6, caractérisé par le fait que la courbe caractéristique est décalée en direction de fréquences de stimulation plus basses (f) lorsque la somme des variations ($\Delta$M) de la valeur de mesure dans deux intervalles successifs d'optimisation (I, II) se situe entre -A1 et +A1.

**8.** Procédé suivant la revendication 6 ou 7, caractérisé par le fait que la courbe caractéristique (K) est décalée vers des fréquences de stimulation plus élevées (f) lorsque la somme des variations ($\Delta$M) de la valeur de mesure dans deux intervalles successifs d'optimisation (I, II) est située entre -A1 et +A1 et qu'une courbe caractéristique limite inférieure (KO) est atteinte.

**9.** Procédé suivant l'une des revendications 6 à 8, caractérisé par le fait que le décalage indiqué de la courbe caractéristique n'est exécuté que pendant une phase de repos du patient.

**10.** Procédé suivant l'une des revendications 1 à 9, caractérisé par le fait que la somme des variations ($\Delta$M) de la valeur de mesure est comparée à une valeur limite positive supérieure (A1) et à une valeur limite inférieure négative (-A1) et que, pendant une phase élevée d'efforts du patient, la valeur limite supérieure de la fréquence de stimulation ($f_{max}$) est modifiée selon le schéma suivant :
a) la valeur limite ($f_{max}$) est réduite lorsque la somme des variations ($\Delta$M) de la valeur de mesure dans au moins un intervalle d'optimisation est inférieure à (-A1) et que la valeur limite ($f_{max}$) n'a pas encore atteint sa valeur prédéterminée la plus basse;
b) la valeur limite ($f_{max}$) reste inchangée lorsque la somme des variations ($\Delta$M) de la valeur de mesure se situe entre (-A1) et (+A1);
c) la valeur limite ($f_{max}$) est accrue lorsque la somme des variations ($\Delta$M) de la valeur de mesure dans au moins un intervalle d'optimisation est supérieure à (A1) et que la valeur limite ($f_{max}$) n'a pas encore atteint sa valeur prédéterminée la plus élevée.

**11.** Procédé suivant la revendication 10, caractérisé par le fait que la valeur limite ($f_{max}$) est réduite lorsque la somme des variations ($\Delta$M) de la valeur de mesure dans deux intervalles d'optimisation successifs (II) se situe entre (-A1) et (+A1) et que la valeur limite ($f_{max}$) n'a pas encore atteint sa valeur prédéterminée la plus basse.

12. Procédé suivant les revendications 10 et 11, caractérisé par le fait que la valeur limite ($f_{max}$) est accrue lorsque la somme des variations ($\Delta M$) de la valeur de mesure pendant deux intervalles d'optimisation successifs (II) se situe entre (-A1) et (+A1) et que la valeur limite ($f_{max}$) a atteint sa valeur prédéterminée la plus basse.

13. Procédé suivant l'une des revendications 1 à 12, caractérisé par le fait que dans le cas d'intervalles d'optimisation (I, II), dans lesquels le nombre, suffisant pour le processus d'optimisation, de variations ($\Delta M$) de la valeur de mesure ne peut pas être obtenu, les variations mesurées de la valeur de mesure sont mémorisées et sont utilisées ultérieurement pour l'intervalle d'optimisation immédiatement suivant.

EP 0 401 265 B1

FIG 1

FIG 2

FIG 3

FIG 4

FIG 5

Fall 2 : $\Sigma\Delta M < -A_1$

Fall 1 : $\Sigma\Delta M > A_1$

$M_{n-2}$  $M_{n-1}$  $M_n$

$\frac{1}{2}(M_n - M_{n-2})$

$M_{n-2}$

$M_{n-1}$  $M_n$

Hohe Last

Ruhe

$f_{max}$

$f_{min}$

$SO_{2\,max}$

$SO_{2\,min}$

$P_{max}$

$P_{min}$

I

II

FIG 6